(19)

Europäisches
Patentamt
European
Patent Office
Office européen
des brevets

(11) EP 3 267 345 A1

(12) **EUROPEAN PATENT APPLICATION**

(43) Date of publication:
10.01.2018 Bulletin 2018/02

(51) Int Cl.:
*G06F 19/12* (2011.01)

(21) Application number: 16178113.3

(22) Date of filing: 06.07.2016

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **European Molecular Biology
Laboratory
69117 Heidelberg (DE)**

(72) Inventors:
• **Jouhten, Paula Tuulia
69115 Heidelberg (DE)**
• **PATIL, Kiran Raosaheb
69120 Heidelberg (DE)**

(74) Representative: **Maiwald Patentanwalts GmbH
Engineering
Elisenhof
Elisenstrasse 3
80335 München (DE)**

(54) **METHODS FOR ADAPTIVE LABORATORY EVOLUTION**

(57)     The invention relates to adaptive laboratory evolution of cells and/or organisms. In particular, the invention relates to a method for adaptive laboratory evolution of cells or organisms in order to generate desired metabolic traits without the need of genetic engineering. In addition, the invention provides a computer program element and a computer readable medium. Further, the invention relates to cells or organisms obtained by the method of the invention.

Figure 1

**Description**

Field of the invention:

The invention relates to adaptive laboratory evolution of cells and/or organisms. In particular, the invention relates to a method for adaptive laboratory evolution of cells or organisms in order to generate desired metabolic traits without the need of genetic engineering. In addition, the invention provides a computer program element and a computer readable medium. Further, the invention relates to cells or organisms obtained by the method of the invention.

Background of the invention:

Adaptive laboratory evolution refers to the culture of cells or organisms under defined conditions leading to adaptive changes that accumulate in populations of cells or (microbial) organisms during selection under specified growth conditions.

Designed modifications of microbial strains have recently achieved increasing interest e.g. in food industry where genetic engineering is often not desired or cannot be applied at all. In addition, even if genetic engineering can be applied, this method is also limited, in particular in the development of complex traits.

So far, it is difficult to predict how the desired traits develop during adaptive laboratory evolution. In particular, the prediction of desired traits under conditions, in which different parameters or components are varied is challenging.

In particular, it is difficult to establish metabolic traits that do not maximize the fitness of the cells. As natural selection favours cells with higher fitness, the selection of conditions for adaptive evolution is non-intuitive, especially when the desired metabolic trait does not maximize the fitness of the cells in the target conditions. The term "target conditions" or "target niche" refers to the chemical environment for the cells or organisms where the desired application, e.g. production of a flavour compound or wine fermentation, is intended.

Consequently, there is a need for a method capable to design modifications of metabolic traits and/or to develop complex metabolic traits. In particular, there is a need for methods to evaluate the suitability of chemical environments to evolve a desired metabolic trait.

Summary of the invention:

It is object of the invention to provide for a method to design chemical environments for adaptive evolution to develop desired metabolic traits.

This object may be solved by the subject-matter according to one of the independent claims. Embodiments of the present invention are described in the dependent claims and with respect to the drawings.

The described embodiments similarly pertain to a method for evaluating the suitability of a chemical environment, a computer program element and a computer readable medium. Synergetic effects may arise from different combinations of the embodiments although they might not be described in detail. It should be noted that in the context of the present invention the terms "flux" and "function of flux" as well as "fluxes" and "functions of fluxes" will be used interchangeably.

Further on, it shall be noted that all embodiments of the present invention concerning a method, might be carried out with the order of the steps as described, nevertheless this has not to be the only and essential order of the steps of the method. All different orders and combinations of the method steps are herewith described.

According to a **first aspect** of the present invention, a method for evaluating the suitability of a chemical environment to evolve a desired metabolic trait of a cell or organism is provided. The method comprises the simulation of one or several fluxes in a metabolic model, in particular the simulation of one or several functions of fluxes in a metabolic model. Thereby, the metabolic model comprises a stoichiometric representation of biochemical reactions and import and export of extracellular compounds. The metabolic trait comprises a set of targets, the latter being functions of fluxes occurring in the cell or organism.

Using the method according to the present invention, a suitable chemical environment can be determined, which can be used to evolve desired metabolic traits of a cell or organism.

According to an embodiment of the invention, a stoichiometric matrix is used in representing the metabolic model. Alternatively, a graph representation of the metabolic model could be provided.

It should be noted that simulation of one or several functions of fluxes in the model in context of the present invention may refer to an optimisation of the one or several functions of fluxes.

A metabolic trait according to the invention is a metabolic characteristic of a cell or organism build by one or a combination of several targets of the corresponding model of the cell or organism. Thus, a metabolic trait is built by at least one target, preferably at least two targets, such as 3, 4, 5, 6, 7, 8, 9, 10 or more targets. A target represents a function of fluxes occurring in the cell or organism.

Typically, a metabolic trait evolves over several generations of the cell or organism. That means that at least two generations, e.g. at least 10, at least 50, at least 100 or more generations of the cell or organism are necessary to evolve a metabolic trait. That means that the cells or organisms are cultured for a certain time in the chemical environment, in particular in the evolution chemical environment, for several days, weeks or months.

The term "metabolic trait" refers to metabolic features of the cell or organisms, e.g. the capability to produce a certain product under certain conditions, the capability to survive and reproduce under certain conditions, such as certain pH, certain gas levels, or certain nutrients, preferably certain gas levels or certain nutrients.

A chemical environment is considered as suitable to evolve a metabolic trait, if the chemical environment is suitable to establish a metabolic trait. That means, when the population of cells or organisms is maintained, i.e. cultured, in the chemical environment, a part of the population of the cell or organism has established the metabolic trait, e.g. that at least one cell or organisms has established the metabolic trait. Preferably, at least 5 %, at least 10 %, at least 20 %, at least 30 %, at least 40 %, at least 50 %, at least 60 %, at least 70 %, at least 80 %, at least 90 % or more of the population have established the metabolic trait.

The term having "established a metabolic trait" means that the metabolic trait will not be lost in the evolution chemical environment in the following generations, such as 2, 10, 100, 1000, 5000 or more generations. In particular, this means that in at least 50%, 60%, 70%, 80 % or 90% of the cells having the metabolic trait, this trait is not lost in the following generations.

In context of the present invention, it might be desirable to have at least one function of flux in the model being an up-regulation target, wherein an up-regulation target is a function of flux for which an increase due to exposure to the chemical environment is desired.

It is a particular advantage of the present invention that the suitability of a chemical environment to evolve a particular metabolic trait of a cell or organism can be rated/evaluated quantitatively. A desired application of the present invention refers to evolution of a cell or organism in an evolution niche with a suitable chemical environment determined within the method. The evolution in the determined suitable chemical environment enforces or enhances a desired metabolic trait. Subsequently transferring the cell or organism into a target niche, e.g. a production chemical environment, the enforced or enhanced metabolic trait of the cell or organism will give rise to a desired product, such as a flavour compound, e.g. produced by yeast.

According to an embodiment of the invention, simulation of one or several targets relative to a growth is performed. Thereby, the growth is a function of fluxes through a reaction or reactions that generate biomass components or biomass.

"Target reactions" or "targets" are considered relative to a growth in order to set or introduce a scale in context of the method. This allows to compare different environments with each other. For instance, the selection pressure on a reaction, i.e. on the activity of a given enzyme, in an adaptive evolution can be considered to be higher, the higher a target is required per unit of growth. This is because natural selection in adaptive evolution favours fast/high-yield growth. For example, in a first environment an up-regulation target has a high value relative to a growth in a cell. In a second environment, however, the value of the same target is lower relative to the growth of the cell, in both cases assuming growth optimality. For an increased growth rate or growth yield, the value of the target in the case of the first environment has to become higher than in the second case. With a comparison relative to growth in each of the two cases, respectively, the two chemical environments can be compared to each other on a quantitative level.

According to an embodiment of the invention, simulation is performed while the growth is constrained to a fixed value or within a range.

Such constraining of the growth further improves the possibility of comparison of different chemical environments.

According to an embodiment of the invention, simulation is performed while a constraint is set on any uptakes in the model of the cell or organism. Thereby, an uptake is a function of a flux or fluxes through a reaction or reactions representing the uptake of extracellular compounds.

The uptake of the extracellular compounds can be active, e.g. mediated by transporters or vesicles, or passive by diffusion of the compound into the cell. Also encompassed is the interaction of compounds with a receptors or enzymes of the cell or organism that influences a metabolic flux.

According to an embodiment of the invention, simulation is performed while a sum of uptakes is constrained to a fixed value or is constrained within a fixed range.

For instance, the sum of uptakes can be constrained to a fixed value obtained by minimizing the sum of uptakes at a fixed growth. Such setting would refer to a situation of optimal or near optimal conversion to growth.

According to an embodiment of the invention, simulation is performed with a sum of absolute values of uptakes (i.e. L1 norm) constrained to an optimal value.

According to an embodiment of the invention, simulation is performed while any of the uptakes are constrained to a fixed value or constrained within a fixed range.

According to an embodiment of the invention, simulation is performed with any of the uptakes constraint to an optimal value. With the term "optimal value" an extreme value may be denoted. These may e.g. be a minimum or maximum value depending on in which direction the reaction is defined in the model.

According to a further embodiment of the present invention, up-regulation targets and down-regulation targets are optimised into opposite directions.

Thereby, an up-regulation target is a target for which an increase is desired and a down-regulation target is a target for which a decrease is desired. An up-regulation target can therefore increase a desired metabolic trait of a cell or organism. On the other hand, down-regulation targets are those targets that can have an undesired, negative or no effect on a desired metabolic trait.

In a simulation of a cell or organism in a particular chemical environment several possible solutions may arise for upregulation and downregulation targets, differing in their performance to enhance the trait. It is the gist of the invention to select for each environment the worst solution(s) to enhance the trait and to use these worst solution(s) for comparing the chemical environments. In order to compare the chemical environments based on their worst performance of enhancing upregulation target(s) and/or supressing downregulation target(s), the absolute values of up-regulation targets can be minimized and the absolute values of the down-regulation targets can be maximised.

According to an embodiment of the invention, within the simulation absolute flux values are considered and simulation is performed using the absolute flux values.

In a metabolic model, the signs of the fluxes depend on the direction in which the fluxes are defined in the model. A negative flux indicates that this flux goes into the opposite direction as a corresponding flux with positive sign.

According to an embodiment of the invention, the number of targets exceeding or falling below at least one threshold is optimized. This optimisation may help to improve the chemical environment.

According to an embodiment of the invention, the number of targets relative to growth exceeding or falling below at least one threshold is simulated.

The number of targets relative to growth beyond a pre-set threshold can give information on the coverage of the targets by the chemical environment.

The measure "coverage of targets" is capable to indicate how many fluxes are optimized in a superior manner compared to reference conditions. The pre-set threshold can be a function of flux or functions of fluxes in the reference conditions. Further, optionally a threshold is applied determining how much higher or lower the values of the targets should be compared to the reference conditions.

According to an embodiment of the invention, at least one threshold is determined with respect to functions of fluxes in a reference chemical environment.

A reference chemical environment can be, in principal, arbitrarily chosen. It is one objective of the method to determine chemical environments that allow better evolution of targets in a cell or organism than evolution of the targets in the cell or organism would be in the reference environment. For example, a reference chemical environment for yeast would be a grape must medium used for growth of the yeast for wine production.

According to a further embodiment of the invention, within the simulation at least one inhibited flux is defined.

According to an embodiment of the invention, simulation is performed by constraining fluxes through reactions, which are targets of inhibitors or regulatory triggers included as components in the chemical environment.

Consequently, according to a further embodiment of the invention, simulation can be performed by constraining those fluxes to zero, which are targets of inhibitors or regulatory triggers included as components in the chemical environment.

According to an embodiment, a method comprises the following additional step of determining a numerical score for the chemical environment. Thereby, the score is indicative for a strength of selection pressure on the targets in the chemical environment. Further, the score is indicative for a coverage of the targets by the selection pressure in the chemical environment.

An explicit example for the definition and determination of a numerical score is provided later on below.

According to an embodiment, a method further comprises the step of determination of a numerical score, wherein the score is indicative for a strength of a worst case selection pressure on the targets in the chemical environment and for a worst-case coverage of the targets by the selection pressure in the chemical environment. According to a further embodiment, a numerical score is defined based on one of the aforementioned embodiments and further takes the number of components in the chemical environment into account.

Possibly, the lower the number of components in the chemical environment, the more cost-efficient the environment can be. Further, it is often not easy to know a priori how different compounds/components are taken up from the chemical environment. This especially applies to the case when several components are provided together. In case of many components it might become less probable that a cell in the chemical environment can take up and use the components in at least close to optimal proportions. In this context, optimal proportions should be understood as the proportions corresponding to optimal growth.

With further reference to a numerical score, in an embodiment of the invention, the score comprises a function of a strength of a selection pressure and a coverage of the targets by the selection pressure as well as a number of components in the chemical environment.

According to an embodiment, a method comprises the following steps: In a first step a first simulation is performed by imposing a plurality of constraints, thereby determining a first simulation result. One of the constraints is understood to set a condition defining a growth to a fixed value or a fixed range. Another constraint sets thermodynamic bounds on the fluxes of the model. Simulation in the first step is performed by optimizing a sum of uptakes. This optimisation is done by minimizing the sum of uptakes of available components of the chemical environment in order to set the optimal conversion of these components to growth of the up taking cell or organism.

In a second method step a second simulation is performed, thereby using or relying on the first simulation result. As a result, a second simulation result is determined.

The second simulation comprises setting a constraint for growth of the cell or organism to a fixed value or, alternatively, constraints the growth within a fixed range. Another constraint, which is set within the second simulation, constraints the sum of uptakes considered in the first simulation to the first simulation result. The second simulation result is indicative for the suitability of the chemical environment and promotes a condition identified as optimizing a sum of up-regulation targets and another condition identified as optimizing a sum of down-regulation targets.

According to a further embodiment, in addition to the aforementioned method steps a third method step is comprised relating to a third simulation, thereby determining a third simulation result. Within this third simulation the number of up-regulation targets enhanced with respect to reference up-regulation targets in a reference chemical environment is optimized. Similarly, a number of down-regulation targets suppressed with respect to down-regulation targets in a reference chemical environment is also optimized within the third simulation.

In a further embodiment of the invention, the determination of the numerical score of a chemical environment is formalised in that the score is given by the following value:

$$value = W_c \cdot \frac{(n - coverage)}{n} + W_s \cdot \frac{(1000 \cdot u - strength)}{n} + W_m \cdot c$$

The formula relies on a simulation comprising the first, the second and the third simulation steps described above. In the above formula, n denotes the number of targets and *coverage* refers to the optimal number of targets obtained within the third simulation step. *u* denotes the number of up-regulation targets, and *strength* is the sum of the optimised sum of up-regulation targets and the optimised sum of down-regulation targets obtained in the second simulation step. With c the number of components in the chemical environment is indicated. Finally, $W_c$, $W_s$ and $W_m$ denote mathematical weights. These weights can be set or chosen by a user. For example, the following values may be chosen for $W_c$, $W_s$ and $W_m$ : 1000, 100, 1. Thereby, assigning the highest weight for the coverage of targets and lowest importance for the number of chemical components in evaluating chemical environments to evolve a trait comprising of the targets. With the choice of specific values for the weights the result from the first, the second and/or the third simulation step - according to the three terms in the sum above - can be given a larger or smaller influence in the numerical score quantified by the numerical value *value.*

The above formula provides an example for the determination of an actual numerical score for a chemical environment and allows a quantitative comparison of different chemical environments, which can be used in order to evolve a desired metabolic trait of a cell or organism. With reference to the detailed description of embodiments, an explicit and simplified sample calculation is given. From this sample calculation the determination of a numerical score according to the above formula will become apparent.

The method of the present invention provides a score evaluating the suitability of a chemical environment. This score can be used to evaluate and rank different chemical environments.

According to a further embodiment, the score evaluating the suitability of chemical environment is calculated using a function involving *strength* and/or *coverage* and/or number of components in the chemical environment. The skilled person will easily adapt this situation without leaving the scope of the invention.

The method of the present invention can be for examples used for fitness scoring in optimization or search algorithms, such as a genetic algorithm or simulated annealing. When a genetic algorithm is used, the chemical environment can for example represent an individual in the genetic algorithm and the presence or absence of compounds of the chemical environment can represent a gene set (sometimes also called traits in the genetic algorithms).

The method of the present invention may also be used to evaluate which natural environments are suitable to evolve a desired metabolic trait. Thus the chemical environment may be a natural environment for a cell or organism. That means that compounds of at least one natural environment that are known or are determined by methods known to the skilled in the art are used for the evaluation of the suitability of the chemical environment. If a natural environment is determined to be suitable to evolve a desired metabolic trait, the organisms can be isolated from the natural environment. The isolated organism can be analysed for the presence of the metabolic trait. Methods for the analysis of the metabolic trait are known to the skilled person and include for example enzymatic tests, metabolite analysis, characterization of the physiology, flux analysis, determining of the proteome or genome of the organism, by methods known to the skilled person (e.g. next generation sequencing).

A metabolic trait may be a metabolic feature of the cell or organism, e.g. the capability to produce a desired product under certain conditions, the capability to survive and reproduce under certain conditions, such as a certain gas levels, the presence or absence of nutrients, or the resistance or sensitivity to a substance. Preferably the metabolic trait allows the cell or organism to produce of at least one desired product by the cell or organism. This means for example that the cell or organism that acquired the metabolic trait is capable to increase the production by 10 %, 20 %, 30 %, 40 %, 50 %, 60 %, 70 %, 80 %, 90 %, 100 %, 150 %, 200 %, 250 %, 300 % or more of the desired product relative to the amount of desired product in the cell or organism before applying the method of present invention. Also contemplated is that the cell or organism is capable of producing the desired compound after acquiring the metabolic trait while it was not capable of producing the compound before it acquired the metabolic trait. The metabolic trait may be for example also the reduced production of a product. For example, the reduced production of alcohol, such as ethanol, in an alcoholic fermentation process.

The at least one desired product may be any product that can be produced by a cell or organism, in particular a microorganism, for example a polymer, an acid, an alcohol or an ester. The at least one desired product may be a food compound, preferably an aroma compound. An aroma compound may be for example alcohol, aldehyde, ester, fatty acid, ketone, lactone, an aromatic compound or pyrazine. Alcohols used as aroma compounds may be for example 1,2-butanediol, 2-butanol, 2-3-butanediol, ethanol, 2-ethylbutanol, 2-ethylhexanol, 2-heptanol, hexanol, isobutanol, 2-methylbutanol, 3-methylbutanol, 2-methylpropanol, 2-nonanol, (Z)-1,5-octadien-3-ol, 2-octanol, 1-octen-3-ol, 1-pentanol, phenylethanol, 2-phenylethanol, 1-nonanol. Examples for aldehydes used as aroma compounds are acetaldehyde, decanal, heptanal, (Z)-4-heptenal, hexanal, 2-hexenal, isohexanal, 2-methylbutanal, 3-methylbutanal, 2-methylpropanal, nonanal, (E,E)-2,4-nonadienal, (Z)-2-nonenal, (E)-2-nonenal, octanal, butanal, pentanal, propanal, propenal, thiophen-2-aldehyde. Examples for esters are methyl acetate, ethyl acetate, ethyl butyrate, ethyl hexonate, ethyl isobutonate, ethyl octanoate, ethyl butanoate, isobutyl butanoate, 2-methyl-1-butyl acetate, 3-methyl-1-butyl acetate, 3-octyl acetate, pentyl acetate, phenethyl acetate, ethyl butyrate, propyl butyrate, 2-hydroxyethyl propionate, 2-methyl-2-ethyl-3-hydroxyhexyl propionate, ethyl 2-methylbutanoate, ethyl 3-methylbutanoate. Examples for fatty acids used as aroma compounds are acetate, butyrate, caproate, decanoate, isobutyrate, 2-methylbutyric acid, 3-methylbutyric acid, octanoate, phenylacetate, propionate, valerate. Examples for lactones used as aroma compounds are $\delta$-decalactone, $\gamma$-decalactone, $\gamma$-butyrolactone, $\delta$-dodecalactone, $\delta$-octalactone, (Z)-6-dodecen-$\delta$-lactone. Examples for ketones used as aroma compounds are acetophenone, acetone, 2,3-butanedione, 2,3-pentandione, 2-butanone, 3-hydroxy-2-butanone, 2-heptanone, 2-hexanone, 3-methyl-2-butanone, 4-methyl-2-pentanone, 2-nonanone, 2-octanone, 1-octen-3-one, 2-pentanone, 3-pentanone, 2-tridecanone, 2-undecanone. Aromatic compounds used as aroma compounds are for example vanillin, benzaldehyde, $\beta$-phenethyl alcohol, trimethylbenzene. Pyrazines used as aroma compounds are for example 2,3-diethyl-5-methylpyrazine, 2-ethyl-3,5-dimethylpyrazine, 2-methoxy-3-isopropylpyrazine.

In preferred embodiments, the cell or organism is of industrial relevance. Particularly contemplated are microorganisms. The microorganism may be selected from the group of bacteria, yeast or molds. An example for an industrial relevant bacterium is *Escherichia,* such as *Escherichia coli.* The yeast may be for example *Saccharomyces,* such as *Saccharomyces cerievisiae.* An example for mold is *Aspergillus,* such as *Aspergillus niger.* Examples of microorganisms that are of industrial relevance are *Pediococcus pentosaceus, Lactobacillus sp. such as Lactobacillus acidophilus, Lactococcus lactis, Lactobacillus plantarum, Lactobacillus delbrueckii subsp. bulgaricus, Streptococcus thermophiles, Acetobacter sp. such as Acetobacter ghanensis, Acetobacter fabarum, Luyveromyces sp. suhc as Kluyveromyces marxianus, Kluyveromyces lactis, Kazachstania exigua, Hansenula polymorpha, Ogataea polymorpha, Chartomium thermophilum, Pichia pastoris, Scheffersomyces stipitis, Issatchenkia orientalis, Torulaspora sp. such as Torulaspora delbrueckii, Schizosaccharomyces pombe, Brettanomyces sp. such as Brettanomyces bruxellensis, Zygosaccharomyces bailii, Ceratocystis fimbriata, Neurospora* sp., such as *Neurospora sitophila, Zygosaccharomyces rouxii, Ceratocystis fimbriata, Lentinus edodes, Neurospora crassa, Phanerochaete* sp., *Trametes* sp., *Sporotrichum thermophile, Vibrio costicola, Rhizopus sp. such as Rhizopus rhizopodiformis, Rhizopus oryzae, Rhizopus oligosporus, Rhizomucor pusillus, Penicillium restriction, Yarrowia lipolytica, Candida rugose, Candida californica, Aspergillus* sp., *such as Aspergillus niger, Aspergillus oryzae, Aspergillus flavipes, Penicillium sp. such as Penicillium simplicisimum, Penicillium citrimum, Penicillium candidum, Penicillium brevicompactum, Bacillus sp.* such as *Bacillus subtilis* or *Bacillus circulans.*

Typically, the chemical environment is a culture medium, also called growth medium, for the cell or organism. The culture medium may be a complex medium, i.e. a medium that contains a mixture of components that are not defined and/or of which the quantities are not defined, such as wort or grape must. Alternatively the culture medium may be a defined medium, i.e. a medium of which all compounds and their quantities are known. Preferably, the culture medium may be a defined medium. Typically, a chemical environment comprises one or more carbon sources (e.g. glucose, ethanol, glycerol, fructose, xylose, lactose, methanol, methane, $CO_2$, arabinose, galactose, gluconic acid, glucuronic acid, ribose, cellulose, hemi-cellulose, pectin, lignin, galacturonic acid, succinic acid, fumaric acid, malic acid, pyruvic acid, fatty acids), one or more nitrogen sources (e.g. peptides, urea, amino acids such as glutamate, aspartate, glutamine, valine, phenylalanine, threonine, serine, amines, $(NH_4)_2SO_4$), trace elements (e.g. $FeSO_4$, $MnCl_2$, $CoCl_2$, $CuSO_4$,

NaMoO$_4$, H$_3$BO$_3$, KI), and vitamins (e.g. pantothenate, thiamine, myo-inositol, nicotinic acid, pyridoxine, biotin, para-amino benzoic acid). The components in a chemical environment may be introduced in different ionic state or different hydrated forms or in different salt forms. The components in a chemical environment may be introduced in different proportions. Carbon source can provide both carbon for biosynthesis and carbon for the generation of energy or there may be separate substrates for biosynthesis and energy. A chemical environment could also consist of several carbon and nitrogen sources or a single or many substrates being both a carbon and nitrogen sources. The chemical environment may comprise different compounds that are useful for evolving a metabolic trait. An exemplary chemical environment may comprise glycerol, an alcohol, as a major carbon source, and L-valine, an amino acid, as a nitrogen source, in addition to several trace elements and vitamins. An exemplary chemical environment may comprise glucose, (NH$_4$)$_2$SO$_4$, KH$_2$PO$_4$, Mg$_2$SO$_4$, L-methionine, FeSO$_4$.7H$_2$O, ZnSO$_4$.7H$_2$O, CaCl$_2$.6H$_2$O, MnCl$_2$.2H$_2$O, CoCl$_2$.6H$_2$O, CuSO$_4$.5H$_2$O, NaMoO$_4$.2H$_2$O, H$_3$BO$_3$, KI and Na$_2$EDTA.2H$_2$O, d-biotin, para-amino benzoic acid, nicotinic acid, Ca-pantothenate, pyridoxine HCL, thiamine HCl and myo-inositol. The chemical environment may further comprise inhibitors such as 1,5-gluconolactone, 2-deoxyglucose, 2-phosphoglycolate, diamide, D-threose 2,4-diphosphate, sodium iodoacetate, 2-phosphoglycolohydroxamate, 4-chloromercuribenzoic acid, 4-Methylpyrazole hydrochloride, 5-phosphoarabinonate, 6-azauracil, 6-azauridine, acetazolamide, acetoacetone, acetopyruvate, acetoacetyl-CoA, allopurinol, aminoethylpyruvate, antimycin A, ascosteroside, echinocandin B der., ergokonin A, aspartate semialdehyde, aureobasidin A, bafilomycin A1, concanamycin A, hygrolidin, lencanicidin , beta-chloro-L-alanine hydrochloride, carmustine, tetraethylthiuram disulfide, thiuram, caspofungin, echinocandin C der., cerulenin C75, clotrimazole, fenpropimorph, cyproconazole, fluconazole, tubulazole, voriconazole, diethylenetriamine pentaacetic acid, eflornithine, etidronate disodium hydrate, pamidronate disodium salt hydrate, fluorouracil, fluvastatin, lescol, glucosamine, hydroxycarbamide, iodoacetamide, leflunomide, methionine sulfoxime phosphate, methotrexate, methylamine, methylmercury, mycophenolate mofetil, mycophenolic acid, myriocin der., myxothiazol A, strobilurin B, niclosamide, nitisinone, nitrate, N-phosphonoacetyl-L-aspartate, oxalate, P1,P5-Di(adenosine-5')pentaphosphate(Ap5A) , pyridoxylalanine, quercetin, rapamycin, sinefungin, soraphen A, TOFA, squalestatin, squalestatin der., zaragozic acid, terbinafine hydrochloride, terbinafine, naftifine, Triacsin C, triclosan, tritoqualine, valproic acid, and vigabatrin.

The target chemical environment is the environment used to culture the cell or organism to exploit the desired metabolic trait. That means that in the target chemical environment the cell or organism is cultured for example to produce a desired product.

In some embodiments, the chemical environment is an evolution chemical environment used to evolve the metabolic trait of the cell or organism. The chemical environment differs from a target chemical environment used for culturing the cell or organism to exploit the metabolic trait, for example to produce at least one desired product. This allows to evolve traits that are not improving the fitness of the cell or organism in the target environment. For example, if, due to the desired metabolic trait (e.g. the production of a desired product) the growth of the cell or organism is reduced in the target chemical environment, the evolution of the metabolic trait in the target chemical environment, in which the metabolic trait is not a fitness advantage, is hardly feasible or not possible at all. Therefore, the cell or organism is first grown in the evolution chemical environment in which at least one of the targets of the metabolic trait provides a fitness advantage. In this case, the method of the invention aims at the evaluation of the suitability of the evolution chemical environment to evolve a metabolic trait.

In an alternative embodiment, the cell may be a tumour cell or a group of tumour cells or a pathogen.

The method of the invention may also be used for the destabilization of the cell or organism. Particularly, the destabilization of a tumour cell or a pathogen is contemplated. Preferably, the destabilization of a tumour cell is contemplated. This means in the context of the invention that the chemical environment would lead to the evolution of metabolic traits that destabilize the cell or organism, e.g. a tumour cell or a pathogen. The destabilization of the cell or organism may be for example a decreased growth and/or death of the cell or organism.

This means that the chemical environment evaluated by the method of the invention might lead to the evolution of a destabilized cell or organism. This means that the destabilized cell or organism is achieved after at least one generation, typically, after several generations of the cell or organism, such as at least 2, at least 3, at least 4, at least 5, at least 10, at least 20, at least 50 or more generations. Preferably, a metabolic trait that destabilizes the cell is achieved after at most 10000 generations, at most 1000 generations, at most 500 generations, at most 300 generations, at most 200 generations, at most 100 generations, at most 50 generations, at most 30 generations, at most 20 generations, at most 10 generations, at most 5 generations, at most 3 generations.

In embodiments, that aim at the destabilization of a cell, different compound compositions (i.e. chemical environments) may be evaluated for the suitability to evolve a metabolic trait that destabilizes the tumour cell, when the composition is administered to a patient suffering from cancer. Thus, the invention also refers to a composition for use as a medicament. In particular, the invention refers a composition for use in treating cancer.

Another aspect of the invention refers to a method for determining a chemical environment to evolve a metabolic trait of a cell or organism comprising the following steps:

(a) Evaluating the suitability of at least two chemical environments using the method as described herein,
(b) Selecting at least one environment with a suitability to evolve a metabolic trait, which exceeds a threshold of suitability.

A further aspect refers to the method providing a chemical environment to evolve a metabolic trait of a cell or organism comprising the following steps:

(a) Evaluating the suitability of at least two chemical environments using the method as described herein,
(b) Selecting at least one chemical environment with a suitability to evolve a metabolic trait, which exceeds a threshold of suitability,
(c) Preparing a chemical environment of step (b).

Another aspect refers to a chemical environment obtainable or obtained by the method described above.

The threshold of suitability may be a pre-set threshold. The threshold of suitability may be set so that the chemical environments exceeding the threshold are the 60%, the 50%, the 40 %, the 30 %, the 20 %, the 10 % with the highest potential to evolve a metabolic trait of all tested chemical environments. In a specific embodiment the environment with the highest potential to evolve a metabolic trait is selected. In another specific embodiment one of the two environments, one of the 3 environments, one of the 4 environments, one of the 5 environments, one of the 6 environments, one of the 10 environments, one of the 20 environments, one of the 50 environments, one of the 100 environments with the highest potential to evolve a metabolic trait is selected.

In addition, the application is concerned with a method for evolving a metabolic trait of the cell or organism comprising the steps:

(a) Evaluating the suitability of at least two chemical environments using the method as described herein,
(b) Selecting at least one environment with a suitability to evolve a metabolic trait, which exceeds a threshold of suitability,
(c) Growing the cell or organism in the chemical environment selected in step (b).

Further, the present application refers to a cell or organism obtained by the methods described herein. In particular, the present application contemplates a cell or organism obtained by a method comprising the following steps:

(a) Evaluating the suitability of at least two chemical environments using the method as described herein,
(b) Selecting at least one environment with a suitability to evolve a metabolic trait, which exceeds a threshold of suitability,
(c) Growing the cell or organism in the chemical environment selected in step (b).

In a specific embodiment, the method for evolving a metabolic trait of the cell or organism comprises the steps,

(a) Evaluating the suitability of at least two evolution chemical environments using the method as described herein,
(b) Selecting at least one evolution chemical environment with a suitability to evolve a metabolic trait, which exceeds a threshold of suitability (b) Growing the cell or organism in the selected evolution chemical environment to evolve the desired trait,
(c) Growing the cell or organism in a target chemical environment to produce at least one desired product by the cell or organism.

According to a further aspect of the invention, a computer program element is provided, which, when it is executed by a processor, is adapted to carry out any or any combination of the method steps described above.

According to another aspect of the invention, a computer readable medium is provided, which comprises the above computer program element.

It may be seen as a gist of the invention to quantify the suitability of a chemical environment to evolve a desired metabolic trait of a cell or organism. With the described method steps, a quantitative comparison of different chemical environments can be performed in order to determine such chemical environments that allow desired evolution of a cell or organism.

It has to be noted that some of the embodiments of the invention are described with reference to different subject-matters. In particular, some embodiments are described with reference to method type claims whereas other embodiments are described with reference to apparatus type claims. However, a person skilled in the art will gather from the above and the following description that unless other notified in addition to any combination of features belonging to one type of subject-matter also any combination between features relating to different subject-matters is considered to be disclosed with this application.

The aspects and embodiments defined above and further aspects, embodiments, features and advantages of the present invention can also be derived from the examples of embodiments to be described hereinafter and are explained with reference to examples of embodiments. The invention will be described in more detail hereinafter with reference to examples of embodiments but to which the invention is not limited.

BRIEF DESCRIPTION OF THE DRAWINGS

Fig. 1 shows a simplified metabolic network and its evolution in an evolution niche (i.e. evolution chemical environment) and a target niche (i.e. target chemical environment).

Fig. 2 shows an illustration of a toy model with exchange reactions for extracellular compounds.

Fig. 3 is an illustration of a genetic algorithm relying on the method of the present invention

DETAILED DESCRIPTION OF EMBODIMENTS

Similar or relating components in the several figures are provided with the same reference numerals. The view in the figure is schematic and not fully scaled.

In the following, a simple toy model example for an algorithm based on the method of the invention is presented. The toy model is meant to provide a simple and comprehensible example. Neither the invention nor one of the embodiments described above is limited to the simple scenario described with respect to the toy model.

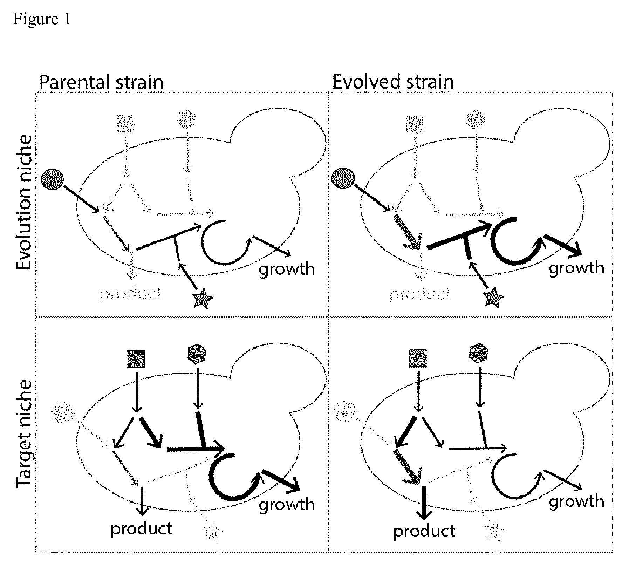

The illustration in Fig. 1 describes with a simplified metabolic network the separation of evolution and target niches, wherein niche is a chemical environment, and thereby the evolution of a metabolic trait that does not provide a fitness benefit in target niche.

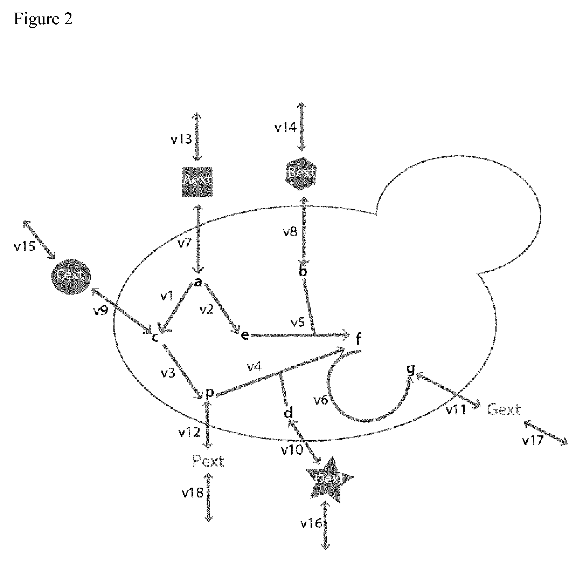

The simplified metabolic network used in the illustration in Fig. 1 is next developed into a metabolic model, which is the toy model considered here used to present an example of calculations based on the method steps of the invention. In Fig. 2 the metabolic network is augmented with exchange reactions for extracellular compounds, referring to import and export of extracellular compounds. Further, the fluxes and metabolites are indicated with identifiers in Fig. 2.

From the reactions indicated in Fig. 2, the following stoichiometric matrix S of the model can be deduced and the following vectors - mass balance vector (b-vector), lower- and upper-bound vectors (lb- and ub-vectors), and biological objective vector (c-vector) are introduced:

S-matrix (the stoichiometric matrix) =

|  | v1 | v2 | v3 | v4 | v5 | v6 | v7 | v8 | v9 | v10 | v11 | v12 | v13 | v14 | v15 | v16 | v17 | v18 |
|---|---|---|---|---|---|---|---|---|---|---|---|---|---|---|---|---|---|---|
| a | -1 | -1 | 0 | 0 | 0 | 0 | -1 | 0 | 0 | 0 | 0 | 0 | 0 | 0 | 0 | 0 | 0 | 0 |
| b | 0 | 0 | 0 | 0 | -1 | 0 | 0 | -1 | 0 | 0 | 0 | 0 | 0 | 0 | 0 | 0 | 0 | 0 |
| c | 1 | 0 | -1 | 0 | 0 | 0 | 0 | 0 | -1 | 0 | 0 | 0 | 0 | 0 | 0 | 0 | 0 | 0 |
| d | 0 | 0 | 0 | -1 | 0 | 0 | 0 | 0 | 0 | -1 | 0 | 0 | 0 | 0 | 0 | 0 | 0 | 0 |
| e | 0 | 1 | 0 | 0 | -1 | 0 | 0 | 0 | 0 | 0 | 0 | 0 | 0 | 0 | 0 | 0 | 0 | 0 |
| f | 0 | 0 | 0 | 1 | 1 | -1 | 0 | 0 | 0 | 0 | 0 | 0 | 0 | 0 | 0 | 0 | 0 | 0 |
| g | 0 | 0 | 0 | 0 | 0 | 1 | 0 | 0 | 0 | 0 | -1 | 0 | 0 | 0 | 0 | 0 | 0 | 0 |
| p | 1 | 0 | 1 | -1 | 0 | 0 | 0 | 0 | 0 | 0 | 0 | -1 | 0 | 0 | 0 | 0 | 0 | 0 |
| aext | 0 | 0 | 0 | 0 | 0 | 0 | 1 | 0 | 0 | 0 | 0 | 0 | -1 | 0 | 0 | 0 | 0 | 0 |
| bext | 0 | 0 | 0 | 0 | 0 | 0 | 0 | 1 | 0 | 0 | 0 | 0 | 0 | -1 | 0 | 0 | 0 | 0 |
| cext | 0 | 0 | 0 | 0 | 0 | 0 | 0 | 0 | 1 | 0 | 0 | 0 | 0 | 0 | -1 | 0 | 0 | 0 |
| dext | 0 | 0 | 0 | 0 | 0 | 0 | 0 | 0 | 0 | 1 | 0 | 0 | 0 | 0 | 0 | -1 | 0 | 0 |
| gext | 0 | 0 | 0 | 0 | 0 | 0 | 0 | 0 | 0 | 0 | 1 | 0 | 0 | 0 | 0 | 0 | -1 | 0 |
| pext | 0 | 0 | 0 | 0 | 0 | 0 | 0 | 0 | 0 | 0 | 0 | 1 | 0 | 0 | 0 | 0 | 0 | -1 |

The following lb- and ub-vectors of the toy model define the flux lower and upper bounds, corresponding to thermodynamic or capacity constraints on the respective flux vector components. Values of- 1000 and 1000 represent, in this sample calculation, unlimited flux bounds:

| lb = | | ub= | |
|---|---|---|---|
| | 0 | | 1000 |
| | 0 | | 1000 |
| | 0 | | 1000 |
| | 0 | | 1000 |
| | 0 | | 1000 |
| | 0 | | 1000 |
| | -1000 | | 1000 |
| | -1000 | | 1000 |
| | -1000 | | 1000 |
| | -1000 | | 1000 |
| | -1000 | | 1000 |
| | -1000 | | 1000 |
| | 0 | | 0 |
| | 0 | | 0 |
| | 0 | | 0 |
| | 0 | | 0 |
| | 0 | | 1000 |
| | 0 | | 1000 |

The following c-vector of the toy model encodes the biological objective of the model:

| |
|---|
| 0 |
| 0 |
| 0 |
| 0 |
| 0 |
| 0 |
| 0 |
| 0 |
| 0 |
| 0 |
| 0 |
| 0 |
| 0 |
| 0 |
| 0 |
| 0 |
| 1 |

(continued)

| 0 |
|---|

Obviously, with the only non-zero component of the above c-vector referring to the flux vector component v17 related to the exchange reaction Gext (see Fig. 2), the biological objective of the toy model considered here is growth creating the extracellular compund Gext.

The following b-vector of the toy model defines the metabolite mass balances, that is balance of the 14 metabolites considered in the toy model. The assumption here is a steady state, such that the components of the b-vector are set to zeros:

| 0 |
|---|
| 0 |
| 0 |
| 0 |
| 0 |
| 0 |
| 0 |
| 0 |
| 0 |
| 0 |
| 0 |
| 0 |
| 0 |
| 0 |

It should be noted that the lb-vector of the toy model introduced above defines a lower bound for each component of a possible flux vector of the toy model. Similarly, the ub-vector of the toy model defined above introduces for each component of a possible flux vector of the toy model an upper bound. In more elaborate scenarios than the toy model considered here, the values for the lower and upper bounds of the components of possible flux vectors may be determined experimentally or may be determined from thermodynamic constraints.

Regarding the c-vector above related to the biological objective, this objective may vary in different models. For instance, in a larger model, the biological objective may not necessarily be growth but for instance a maximization of ATP generation.

Finally, simulation of a steady state, incorporated with a b-vector with all components set to zero as indicated for the case of the toy model above, is one convenient way to simulate evolution of a cell or organism in a chemical environment, thereby assuming that the system is in a steady-state characterised by the above described mass balance.

A quantitative determination of the suitability of a chemical environment according to an embodiment of the invention is based on the knowledge of and the subsequent comparison with a reference situation. In context of the invention, a comparison to a reference scenario is intended in order to quantitatively rate the suitability or potential of a chemical environment. In particular, with respect to consideration of a worst-case coverage according to the invention, reference state flux ranges may be considered and compared to fluxes in the respective model. Accordingly, in the toy model calculation, reference state fluxes are needed in context of consideration of a worst-case coverage according to the invention.

The worst-case coverage and the calculation associated therewith is considered later on. Here, the reference state for the toy model calculation is defined to be a state with growth on the substrates A and B, compare Fig. 2.

The reference state is described by the minimum and maximum values of each of the fluxes as following:

|    | min | max |
|----|-----|-----|
| v1 | 0   | 0   |

(continued)

|  | min | max |
|---|---|---|
| v2 | 10 | 10 |
| v3 | 0 | 0 |
| v4 | 0 | 0 |
| v5 | 10 | 10 |
| v6 | 10 | 10 |
| v7 | -10 | -10 |
| v8 | -10 | -10 |
| v9 | 0 | 0 |
| v10 | 0 | 0 |
| v11 | 10 | 10 |
| v12 | 0 | 0 |
| v13 | -10 | -10 |
| v14 | -10 | -10 |
| v15 | 0 | 0 |
| v16 | 0 | 0 |
| v17 | 10 | 10 |
| v18 | 0 | 0 |

After this preparation, method steps according to embodiments of the invention are now explained with reference to the toy model. To this end, the suitability or potential of a chemical environment containing substrates C and D to up-regulate flux through reaction v3 (i.e. v3 is the up-regulation target) shall be assessed.

It should be noted that in order to simplify the situation, no inhibition is considered within the toy model.

In a first step, a growth optimality condition is set using linear optimization as follows:

$$\min \sum v_{uptake}, \qquad (1)$$

subject to the following constraints:

$$S \cdot v = 0, \qquad (2)$$

$$v_{\mu} = 10, \qquad (3)$$

$$v_{inhibited} = 0, \qquad (4)$$

$$v_{lb} \leq v \leq v_{ub}. \qquad (5)$$

Here $v_{uptake}$ denote the fluxes of the uptake reactions of the components available in the particular chemical environment, S denotes the stoichiometric matrix of the genome-scale metabolic toy model of the species, $v$ denotes a flux vector, $v_{\mu}$ is the growth rate (i.e. flux), $v_{inhibited}$ are the fluxes through the reactions inhibited by the inhibitors present in the particular chemical environment, $v_{lb}$ and $v_{ub}$ the flux lower and upper bounds, respectively.

It is noted that in most models the uptake fluxes are defined negative, such that in these situations the above minimization would be done on -$\nu_{uptake}$. The skilled person will easily adapt this situation without leaving the scope of the invention.

The above prescriptions given in equations (1) to (5) are implemented into the following form:

$$\max f'x \qquad (I)$$

subject to the constraints

$$Aeq \cdot x = beq, \qquad (II)$$

$$lb_i \le x_i \le ub_i, \qquad (III)$$

where the above introduced quantities Aeq, lb, ub, f and beq are given as follows:

The matrix Aeq is given by the S-matrix of the toy model with two columns added, which refer to possible secretion of the components of the chemical environment.

This refers to the constraints in equation 2 above. Accordingly, Aeq is given by:

| -1 | -1 | 0  | 0  | 0  | 0  | -1 | 0  | 0  | 0  | 0  | 0  | 0  | 0  | 0  | 0  | 0  | 0  | 0  | 0  |
|----|----|----|----|----|----|----|----|----|----|----|----|----|----|----|----|----|----|----|----|
| 0  | 0  | 0  | 0  | -1 | 0  | 0  | -1 | 0  | 0  | 0  | 0  | 0  | 0  | 0  | 0  | 0  | 0  | 0  | 0  |
| 1  | 0  | -1 | 0  | 0  | 0  | 0  | 0  | -1 | 0  | 0  | 0  | 0  | 0  | 0  | 0  | 0  | 0  | 0  | 0  |
| 0  | 0  | 0  | -1 | 0  | 0  | 0  | 0  | 0  | -1 | 0  | 0  | 0  | 0  | 0  | 0  | 0  | 0  | 0  | 0  |
| 0  | 1  | 0  | 0  | -1 | 0  | 0  | 0  | 0  | 0  | 0  | 0  | 0  | 0  | 0  | 0  | 0  | 0  | 0  | 0  |
| 0  | 0  | 0  | 1  | 1  | -1 | 0  | 0  | 0  | 0  | 0  | 0  | 0  | 0  | 0  | 0  | 0  | 0  | 0  | 0  |
| 0  | 0  | 0  | 0  | 0  | 0  | 0  | 0  | 0  | 0  | -1 | 0  | 0  | 0  | 0  | 0  | 0  | 0  | 0  | 0  |
| 1  | 0  | -1 | 0  | 0  | 0  | 0  | 0  | -1 | 0  | 0  | 0  | 0  | 0  | 0  | 0  | 0  | 0  | 0  | 0  |
| 0  | 0  | 1  | -1 | 0  | 0  | 1  | 0  | 0  | 0  | 0  | -1 | -1 | 0  | 0  | 0  | 0  | 0  | 0  | 0  |
| 0  | 0  | 0  | 0  | 0  | 0  | 0  | 1  | 0  | 0  | 0  | 0  | 0  | -1 | 0  | 0  | 0  | 0  | 0  | 0  |
| 0  | 0  | 0  | 0  | 0  | 0  | 0  | 0  | 1  | 0  | 0  | 0  | 0  | 0  | -1 | 0  | 0  | 0  | -1 | 0  |
| 0  | 0  | 0  | 0  | 0  | 0  | 0  | 0  | 0  | 1  | 0  | 0  | 0  | 0  | 0  | 1  | 0  | 0  | 0  | -1 |
| 0  | 0  | 0  | 0  | 0  | 0  | 0  | 0  | 0  | 0  | 1  | 0  | 0  | 0  | 0  | 0  | -1 | 0  | 0  | 0  |
| 0  | 0  | 0  | 0  | 0  | 0  | 0  | 0  | 0  | 0  | 0  | 1  | 0  | 0  | 0  | 0  | 0  | -1 | 0  | 0  |

The vectors lb, ub and f introduced in equations (I) to (III) are given by:

| lb= | | ub= | | f= | |
|---|---|---|---|---|---|
| | 0 | | 1000 | | 0 |
| | 0 | | 1000 | | 0 |
| | 0 | | 1000 | | 0 |
| | 0 | | 1000 | | 0 |
| | 0 | | 1000 | | 0 |
| | 0 | | 1000 | | 0 |
| | -1000 | | 1000 | | 0 |
| | -1000 | | 1000 | | 0 |
| | -1000 | | 1000 | | 0 |
| | -1000 | | 1000 | | 0 |
| | -1000 | | 1000 | | 0 |
| | -1000 | | 1000 | | 0 |
| | 0 | | 0 | | 0 |
| | 0 | | 0 | | 0 |
| | -1000 | | 0 | | 1 |
| | -1000 | | 0 | | 1 |
| | 10 | | 10 | | 0 |
| | 0 | | 1000 | | 0 |
| | 0 | | 1000 | | 0 |
| | 0 | | 1000 | | 0 |

The last three rows of lb, ub and f represent the constraints for the possible secretion of the components of the chemical environment, which refers to the constraints in Equation 3.

The value 1 in vector f above represent the uptakes of the components of chemical environment in the objective, corresponding to constraints in Equation 1.

The 5th and the 6th last row of lb and up represent the free uptakes of the components of chemical environment allowed.

The following vector beq represents the b-vector of the toy model, corresponding to constraints in Equation 2:

| |
|---|
| 0 |
| 0 |
| 0 |
| 0 |
| 0 |
| 0 |
| 0 |
| 0 |
| 0 |
| 0 |
| 0 |
| 0 |
| 0 |

(continued)

| 0 |
|---|

The above system of equations is solved with, for instance, the CPLEX LP-solver or GLPK solver. The solver returns values of the variables corresponding to the optimal value of the objective function. It is worth to note that x may not be unique in real cases. However, the optimal value of objective function (i.e. *f'x)* is unique. In the toy model considered here, the following - in this case unique - values for the components of x are obtained:

| |
|---|
| 0 |
| 0 |
| 10 |
| 10 |
| 0 |
| 10 |
| 0 |
| 0 |
| -10 |
| -10 |
| 10 |
| 0 |
| 0 |
| 0 |
| -10 |
| -10 |
| 10 |
| 0 |
| 0 |
| 0 |

In the next method step in the toy model calculation, the simulation of worst-case selection pressure on the target reactions under the growth-optimality constraint can be considered according to the equations:

$$min \sum |v_{up}| + \sum -|v_{down}|, \tag{6}$$

subject to

$$S \cdot v = 0, \tag{7}$$

$$v_\mu = 10, \tag{8}$$

$$v_{inhibited} = 0, \tag{9}$$

$$v_{lb} \leq v \leq v_{ub}, \qquad\qquad (10)$$

$$\sum v_{uptake} \leq min \sum v_{uptake}. \qquad\qquad (11)$$

Here, $v_{up}$ and $v_{down}$ are fluxes through the up- and down-regulation target reactions, respectively.

In the particular example of the toy model considered here, the target flux is not reversible. Therefore, there is no need to add variables and constraints to implement the optimization of the absolute values of fluxes as a mixed-integer linear programming problem here. However, in case of more elaborate scenarios, this might be necessary.

The above constraints are brought into the following form:

$$\max f'x \qquad\qquad (IV)$$

subject to

$$Aeq \cdot x = beq, \qquad\qquad (V)$$

$$A \cdot x \leq b, \qquad\qquad (VI)$$

$$lb_i \leq x_i \leq ub_i. \qquad\qquad (VII)$$

The matrix Aeq in equation (IV) is given by the S-matrix of the model with two columns for flux variables added to describe the possible secretion of the components of the chemical environment. This corresponds to the constraints in equation 7 above. Accordingly, the matrix Aeq reads:

| | | | | | | | | | | | | | | | | | | | |
|---|---|---|---|---|---|---|---|---|---|---|---|---|---|---|---|---|---|---|---|
| -1 | -1 | 0 | 0 | 0 | 0 | -1 | 0 | 0 | 0 | 0 | 0 | 0 | 0 | 0 | 0 | 0 | 0 | 0 | 0 |
| 0 | 0 | 0 | 0 | -1 | 0 | 0 | -1 | 0 | 0 | 0 | 0 | 0 | 0 | 0 | 0 | 0 | 0 | 0 | 0 |
| 1 | 0 | -1 | 0 | 0 | 0 | 0 | 0 | -1 | 0 | 0 | 0 | 0 | 0 | 0 | 0 | 0 | 0 | 0 | 0 |
| 0 | 0 | 0 | -1 | 0 | 0 | 0 | 0 | 0 | -1 | 0 | 0 | 0 | 0 | 0 | 0 | 0 | 0 | 0 | 0 |
| 0 | 1 | 0 | 0 | -1 | 0 | 0 | 0 | 0 | 0 | 0 | 0 | 0 | 0 | 0 | 0 | 0 | 0 | 0 | 0 |
| 0 | 0 | 0 | 1 | 1 | -1 | 0 | 0 | 0 | 0 | 0 | 0 | 0 | 0 | 0 | 0 | 0 | 0 | 0 | 0 |
| 0 | 0 | 0 | 0 | 0 | 1 | 0 | 0 | 0 | 0 | -1 | 0 | 0 | 0 | 0 | 0 | 0 | 0 | 0 | 0 |
| 1 | 0 | -1 | 0 | 0 | 0 | 0 | 0 | -1 | 0 | 0 | 0 | 0 | 0 | 0 | 0 | 0 | 0 | 0 | 0 |
| 0 | 0 | 1 | -1 | 0 | 0 | 1 | 0 | 0 | 0 | 0 | -1 | -1 | 0 | 0 | 0 | 0 | 0 | 0 | 0 |
| 0 | 0 | 0 | 0 | 0 | 0 | 0 | 1 | 0 | 0 | 0 | 0 | 0 | -1 | 0 | 0 | 0 | 0 | 0 | 0 |
| 0 | 0 | 0 | 0 | 0 | 0 | 0 | 0 | 1 | 0 | 0 | 0 | 0 | 0 | -1 | 0 | 0 | 0 | -1 | 0 |
| 0 | 0 | 0 | 0 | 0 | 0 | 0 | 0 | 0 | 1 | 0 | 0 | 0 | 0 | 0 | -1 | 0 | 0 | 0 | -1 |
| 0 | 0 | 0 | 0 | 0 | 0 | 0 | 0 | 0 | 0 | 1 | 0 | 0 | 0 | 0 | 0 | -1 | 0 | 0 | 0 |
| 0 | 0 | 0 | 0 | 0 | 0 | 0 | 0 | 0 | 0 | 0 | 1 | 0 | 0 | 0 | 0 | 0 | -1 | 0 | 0 |

The constraints introduced by equation 8 and equation 11 referring to quantity A in equation (VI) can be written as

| | | | | | | | | | | | | | | | | | | | |
|---|---|---|---|---|---|---|---|---|---|---|---|---|---|---|---|---|---|---|---|
| 0 | 0 | 0 | 0 | 0 | 0 | 0 | 0 | 0 | 0 | 0 | 0 | 0 | 0 | 0 | 0 | -1 | 0 | 0 | 0 |
| 0 | 0 | 0 | 0 | 0 | 0 | 0 | 0 | 0 | 0 | 0 | 0 | 0 | 0 | -1 | -1 | 0 | 0 | 0 | 0 |

where the first line refers to the constraint from equation 8 and the second line to the constraint from equation 11.

Further, the following vectors lb, up and f arise in this method step within the toy model:

| lb = | ub= | f= |
|---|---|---|
| 0 | 1000 | 0 |
| 0 | 1000 | 0 |
| 0 | 1000 | 1 |
| 0 | 1000 | 0 |
| 0 | 1000 | 0 |
| 0 | 1000 | 0 |
| -1000 | 1000 | 0 |
| -1000 | 1000 | 0 |
| -1000 | 1000 | 0 |
| -1000 | 1000 | 0 |
| -1000 | 1000 | 0 |
| -1000 | 1000 | 0 |
| 0 | 0 | 0 |
| 0 | 0 | 0 |
| -1000 | 0 | 0 |
| -1000 | 0 | 0 |
| 10 | 10 | 0 |
| 0 | 1000 | 0 |
| 0 | 1000 | 0 |
| 0 | 1000 | 0 |

From the constraint in equation 7 the following b-vector beq results:

| |
|---|
| 0 |
| 0 |
| 0 |
| 0 |
| 0 |
| 0 |
| 0 |
| 0 |
| 0 |
| 0 |
| 0 |
| 0 |
| 0 |
| 0 |

Finally, the constrains from equations 8 and 11 translated in the form of equation (VI) give rise to the following value of b in equation (VI):

| |
|---|
| -10 |
| 20 |

Again, the problem is solved with, for instance, either a CPLEX LP or CPLEX MILP solver depending on whether there were reversible fluxes (i.e. fluxes with a lower bound smaller than 0, lb < 0) involved in the targets. In the toy model case the solver returns as *x* (i.e. values of the variables) corresponding to the optimal value of the objective function the following vector x:

| |
|---|
| 0 |
| 0 |
| 10 |
| 10 |
| 0 |
| 10 |
| 0 |
| 0 |
| -10 |
| -10 |
| 10 |
| 0 |
| 0 |
| 0 |
| -10 |
| -10 |
| 10 |
| 0 |
| 0 |
| 0 |

It should again be noted that x may not be unique in real cases, only the optimal value of objective function (i.e. *f'x*) is unique.

According to the above result, the optimal value of the objective function (i.e. *f'x*), which is related to the score of the strength of the selection pressure created by the particular chemical environment, is given by 10 in the toy model case considered here.

Next, the simulation of a worst-case target reaction coverage under the growth-optimality and worst-case selection pressure constraints can be performed within the toy model as follows:

$$\min \sum b_{targets} \tag{12}$$

subject to

$$S \cdot v = 0, \tag{13}$$

$$v_\mu = 10, \tag{14}$$

$$v_{inhibited} = 0\,, \tag{15}$$

$$v_{lb} \leq v \leq v_{ub}, \tag{16}$$

$$\sum v_{uptake} \leq min \sum v_{uptake}\ \,, \tag{17}$$

$$\sum \left|v_{up}\right| + \sum -\left|v_{down}\right| \leq min\left(\sum\left|v_{up}\right| + \sum -\left|v_{down}\right|\right), \tag{18}$$

$$\left|v_{up}\right| - \left((1+\delta)\cdot w_{ub} - \left|v_{up}\right|_{ub}\right)\cdot\left(1 - b_{targets}\right) \leq \left|v_{up}\right|_{ub}, \tag{19}$$

$$\left|v_{down}\right| - \left((1-\delta)\cdot w_{lb} - \left|v_{down}\right|_{lb}\right)\cdot\left(1 - b_{targets}\right) \geq \left|v_{down}\right|_{lb}, \tag{20}$$

$$b_{targets}\epsilon\{0,1\}. \tag{21}$$

Here, $b_{targets}$ are binary variables defining whether the absolute fluxes through the target reactions are beyond a corresponding thresholds defined from absolute flux ranges under reference conditions (i.e. $w_{lb}$, $w_{ub}$) or not. The case of the reference scenario with its reference conditions for the toy model calculation has been considered above and the values obtained for this scenario will now be used in the toy model calculation.

The parameter $\delta$ in equations (19) and (20) above is a threshold parameter stating how much lower or higher a flux relative to growth should be in the particular chemical environment in order to consider the corresponding target reaction to become exposed to (i.e. covered by) the selection pressure. Accordingly, by equations (19) and (20), an example for quantitative determination of a coverage of the target reaction in the toy scenario is introduced. Together with the constraints from equation (18), a worst-case coverage scenario is therefore considered by the set of equations (12) to (21).

For explicit calculation, the above constraints are brought into the following form, where x may contain both continuous and integer variables:

$$\max f'x \tag{VIII}$$

subject to

$$Aeq \cdot x = beq, \tag{IX}$$

$$A \cdot x \leq b, \tag{X}$$

$$lb_i \leq x_i \leq ub_i, \tag{XI}$$

$$x = [x_b, x_c], \tag{XII}$$

$$x_b \in \{0,1\}. \tag{XIII}$$

The quantities in the equations (VIII) to (XIII) above are given by the following expressions.

Aeq represents the S-matrix of the model again with two columns added to describe the possible secretion of the components of the chemical environment, and with an additional column added for a coverage variable of the target reaction, reflecting the constraints in equation 13. Accordingly, Aeq in equation (IX) reads:

| | | | | | | | | | | | | | | | | | | | | |
|---|---|---|---|---|---|---|---|---|---|---|---|---|---|---|---|---|---|---|---|---|
| -1 | -1 | 0 | 0 | 0 | 0 | -1 | 0 | 0 | 0 | 0 | 0 | 0 | 0 | 0 | 0 | 0 | 0 | 0 | 0 | 0 |
| 0 | 0 | 0 | 0 | -1 | 0 | 0 | -1 | 0 | 0 | 0 | 0 | 0 | 0 | 0 | 0 | 0 | 0 | 0 | 0 | 0 |
| 1 | 0 | -1 | 0 | 0 | 0 | 0 | 0 | -1 | 0 | 0 | 0 | 0 | 0 | 0 | 0 | 0 | 0 | 0 | 0 | 0 |
| 0 | 0 | 0 | -1 | 0 | 0 | 0 | 0 | 0 | 1 | 0 | 0 | 0 | 0 | 0 | 0 | 0 | 0 | 0 | 0 | 0 |
| 0 | 1 | 0 | 0 | -1 | 0 | 0 | 0 | 0 | 0 | 0 | 0 | 0 | 0 | 0 | 0 | 0 | 0 | 0 | 0 | 0 |
| 0 | 0 | 0 | 1 | 1 | -1 | 0 | 0 | 0 | 0 | 0 | 0 | 0 | 0 | 0 | 0 | 0 | 0 | 0 | 0 | 0 |
| 0 | 0 | 0 | 0 | 0 | 1 | 0 | 0 | 0 | 0 | -1 | 0 | 0 | 0 | 0 | 0 | 0 | 0 | 0 | 0 | 0 |
| 1 | 0 | -1 | 0 | 0 | 0 | 0 | 0 | -1 | 0 | 0 | 0 | 0 | 0 | 0 | 0 | 0 | 0 | 0 | 0 | 0 |
| 0 | 0 | 1 | -1 | 0 | 0 | 1 | 0 | 0 | 0 | 0 | -1 | -1 | 0 | 0 | 0 | 0 | 0 | 0 | 0 | 0 |
| 0 | 0 | 0 | 0 | 0 | 0 | 0 | 1 | 0 | 0 | 0 | 0 | 0 | -1 | 0 | 0 | 0 | 0 | 0 | 0 | 0 |
| 0 | 0 | 0 | 0 | 0 | 0 | 0 | 0 | 1 | 0 | 0 | 0 | 0 | 0 | -1 | 0 | 0 | 0 | -1 | 0 | 0 |
| 0 | 0 | 0 | 0 | 0 | 0 | 0 | 0 | 0 | 1 | 0 | 0 | 0 | 0 | 0 | -1 | 0 | 0 | 0 | -1 | 0 |
| 0 | 0 | 0 | 0 | 0 | 0 | 0 | 0 | 0 | 0 | 1 | 0 | 0 | 0 | 0 | 0 | -1 | 0 | 0 | 0 | 0 |
| 0 | 0 | 0 | 0 | 0 | 0 | 0 | 0 | 0 | 0 | 0 | 1 | 0 | 0 | 0 | 0 | 0 | -1 | 0 | 0 | 0 |

The quantity A in equation (X) above encodes the constraints in equations 14, 17 and 19, and is therefore given by:

| | | | | | | | | | | | | | | | | | | | | |
|---|---|---|---|---|---|---|---|---|---|---|---|---|---|---|---|---|---|---|---|---|
| 0 | 0 | 0 | 0 | 0 | 0 | 0 | 0 | 0 | 0 | 0 | 0 | 0 | 0 | 0 | 0 | -1 | 0 | 0 | 0 | 0 |
| 0 | 0 | 0 | 0 | 0 | 0 | 0 | 0 | 0 | 0 | 0 | 0 | 0 | 0 | -1 | -1 | 0 | 0 | 0 | 0 | 0 |
| 0 | 0 | 1 | 0 | 0 | 0 | 0 | 0 | 0 | 0 | 0 | 0 | 0 | 0 | 0 | 0 | 0 | 0 | 0 | 0 | -1000 |
| 0 | 0 | 1 | 0 | 0 | 0 | 0 | 0 | 0 | 0 | 0 | 0 | 0 | 0 | 0 | 0 | 0 | 0 | 0 | 0 | 0 |

The vectors lb, ub, and f are given by the following expressions:

| lb = | | ub= | | f= | |
|---|---|---|---|---|---|
| | 0 | | 1000 | | 0 |
| | 0 | | 1000 | | 0 |
| | 0 | | 1000 | | 0 |
| | 0 | | 1000 | | 0 |
| | 0 | | 1000 | | 0 |
| | 0 | | 1000 | | 0 |
| | -1000 | | 1000 | | 0 |
| | -1000 | | 1000 | | 0 |
| | -1000 | | 1000 | | 0 |
| | -1000 | | 1000 | | 0 |
| | -1000 | | 1000 | | 0 |

(continued)

| lb = | 0 | ub= | 1000 | f= | 0 |
|---|---|---|---|---|---|
| | -1000 | | 1000 | | 0 |
| | 0 | | 0 | | 0 |
| | 0 | | 0 | | 0 |
| | -1000 | | 0 | | 0 |
| | -1000 | | 0 | | 0 |
| | 10 | | 10 | | 0 |
| | 0 | | 1000 | | 0 |
| | 0 | | 1000 | | 0 |
| | 0 | | 1000 | | 0 |
| | 0 | | 1 | | 1 |

Further, the following variable types (C= continuous; B= binary) for the components of flux vectors in this calculation step are defined to be:

| |
|---|
| C |
| C |
| C |
| C |
| C |
| C |
| C |
| C |
| C |
| C |
| C |
| C |
| C |
| C |
| C |
| C |
| C |
| C |
| C |
| C |
| B |

The constraints from equation 13 are represented by the following vector beq:

| |
|---|
| 0 |

(continued)

| |
|---|
| 0 |
| 0 |
| 0 |
| 0 |
| 0 |
| 0 |
| 0 |
| 0 |
| 0 |
| 0 |
| 0 |
| 0 |
| 0 |

Finally, the quantity b in equation (X), representing constraints in equations 14, 17 and 19 is given by:

| |
|---|
| -10 |
| 20 |
| 0 |
| 10 |

The problem as given by the equations (VIII) to (XIII) is then solved with e.g. the CPLEX MILP solver.
In case of the toy model the solver returns as $x$ (i.e. values of the variables) corresponding to the optimal value of the objective function the following vector:

| |
|---|
| 0 |
| 0 |
| 10 |
| 10 |
| 0 |
| 10 |
| 2.27E-13 |
| 0 |
| -10 |
| -10 |
| 10 |
| 2.27E-13 |
| 0 |
| 0 |
| -10 |
| -10 |

(continued)

| |
|---|
| 10 |
| 2.27E-13 |
| 0 |
| 0 |
| 1 |

Thus, the optimal value of the objective function (i.e. $f'x$), which corresponds to the score of the coverage of the targets by the selection pressure created by the particular chemical environment, is 1 (the value in the last component of the above vector). This value implies that the only target in this case is covered even in worst-case.

Accordingly, in the toy model case, the chemical environment containing C and D is expected to create stronger selection pressure on the increase of the target flux than the reference chemical environment.

A combined numerical score can be derived from the worst-case selection pressure, the worst-case coverage, and the number of components in the particular chemical environment:

$$value = W_c \cdot \frac{(n - coverage)}{n} + W_s \cdot \frac{(1000 \cdot u - strength)}{n} + W_m \cdot c \qquad (22)$$

*value* is the combined score (i.e. the lower the value the better the particular chemical environment is to evolve the trait), n is the number of target reactions, coverage is the worst-case coverage (i.e. min $\sum b_{targets}$), u is the number of up-regulation target reactions, strength is the worst-case selection pressure strength (i.e. $min \sum |v_{up}| + \sum -|v_{down}|$), c is the number of components in the particular chemical environment, and $W_c$, $W_s$, $W_m$ are weights to be assigned for the coverage, strength, and the complexity of the chemical environment, respectively.

In the toy model case, setting the weight to $W_c$ = 1000, $W_s$ = 1, and $W_m$ = 1, the combined score of the chemical environment containing compounds C and D is obtained to be 992.

If several chemical environments were evaluated, this score would then be compared against the scores of the other chemical environments to choose a suitable chemical environment out of the set of considered chemical environments. Generally, the lower the score the better suited is the particular chemical environment to evolve a particular desired metabolic trait. The skilled person will easily adapt the scoring formula, for example so that the higher the score the better the suitability, without leaving the scope of the invention.

The design of the chemical environment could also be carried out by performing optimization of the combination of components from a set of possible components e.g. using the method according to the invention as a fitness function in a genetic algorithm.

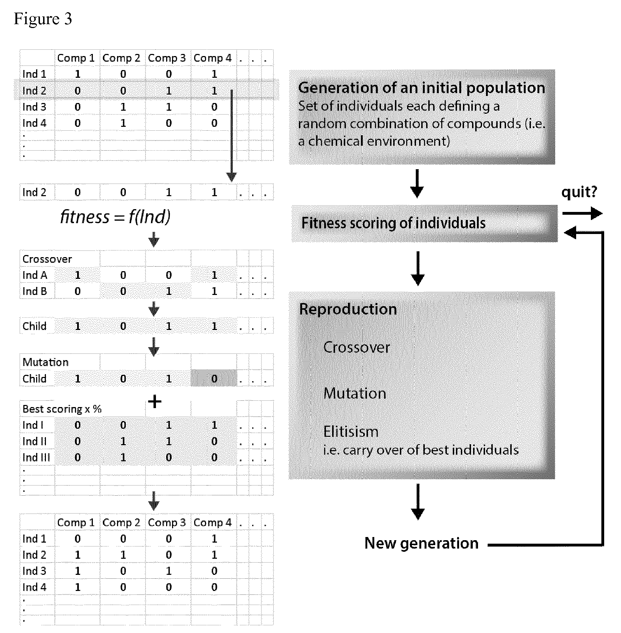

With respect to Fig. 3 an example for an application of a method according to the invention in context of a genetic algorithm is given. The algorithm is suited for the design of optimal chemical environments for an adaptive evolution of a metabolic trait. Individuals of the population in the genetic algorithm are particular chemical environments, defined as binary vectors representing the combinations of compounds. The fitness scoring, evaluates the goodness of an individual chemical environment for adaptive evolution of the metabolic trait. Reproduction phase creates a new generation of individuals through crossovers, mutations, and elitism.

The application also comprises the following embodiments:

Embodiment 1: A method for evaluating the suitability of a chemical environment to evolve a desired metabolic trait of a cell or organism, the method comprising simulation of one or several of functions of fluxes in a metabolic model, wherein the metabolic model comprises a stoichiometric representation of biochemical reactions and import and export of extracellular compounds,
wherein the metabolic trait comprises a set of targets,
wherein the targets are functions of fluxes.

Embodiment 2: A method according to embodiment 1, wherein simulation of one or several targets relative to growth is performed,
wherein the growth is a function of fluxes through a reaction or reactions that generate biomass components or biomass.

Embodiment 3: A method according to embodiment 2,

wherein a simulation is performed while the growth is constrained to a fixed value or within a range.

Embodiment 4: A method according to embodiment 3,
wherein a simulation is performed while the growth is constrained to its optimal value.

Embodiment 5: A method according to embodiment 3 or 4, wherein simulation is performed while a constraint is set on any of uptakes,
wherein uptakes are functions of fluxes through the reactions representing the import of extracellular compounds.

Embodiment 6: A method according to embodiment 5, wherein a simulation is performed while a sum of uptakes is constrained to a fixed value or within a range.

Embodiment 7: A method according to embodiment 6,
wherein a simulation is performed while a sum of uptakes is constrained to an optimal value.

Embodiment 8: A method according to embodiment 7,
wherein a simulation is performed while any of uptakes is constrained to a fixed value or within a range.

Embodiment 9: A method according to embodiment 8,
wherein a simulation is performed while any of uptakes is constrained to an optimal value.

Embodiment 10: A method according to any of the preceding embodiments, wherein up-regulation targets and down-regulation targets are optimized into opposite directions,
wherein the up-regulation targets are those targets for which an increase is desired, and
wherein the down-regulation targets are those targets for which a decrease is desired.

Embodiment 11: A method according to any of the preceding embodiments,
wherein simulation of absolute flux values is performed.

Embodiment 12: A method according to any of the preceding embodiments,
wherein absolute values of up-regulation targets are minimized and absolute values of down-regulation targets are maximized.

Embodiment 13: A method according to any of the preceding embodiments,
wherein the number of targets exceeding or falling below at least one threshold is optimized.

Embodiment 14: A method according to any of the preceding embodiments,
wherein the number of targets relative to a growth exceeding or falling below at least one threshold is simulated.

Embodiment 15: A method according to embodiment 13 or 14,
wherein the at least one threshold is determined with respect to functions of fluxes in a reference chemical environment.

Embodiment 16: A method according to any of the preceding embodiments,
wherein in the simulation at least one inhibited flux is defined.

Embodiment 17: A method according to any of the preceding embodiments,
wherein simulation is performed by constraining fluxes through reactions that are targets of inhibitors or regulatory triggers included in the chemical environment.

Embodiment 18: A method according to any of the preceding embodiments,
wherein simulation is performed by constraining to zero fluxes through reactions that are targets of inhibitors or regulatory triggers included in the chemical environment.

Embodiment 19: A method according to any of the preceding embodiments,
wherein a stoichiometric matrix of the metabolic model is used.

Embodiment 20: A method according to any of the preceding embodiments, further comprising: determination of a numerical score for the chemical environment,
wherein the score is indicative for a strength of selection pressure on the targets in the chemical environment, and/or
wherein the score is indicative for a coverage of the targets by the selection pressure in the chemical environment.

Embodiment 21: A method according to any of the preceding embodiments, further comprising: determination of a numerical score for the chemical environment,
wherein the score is indicative for a strength of a worst-case selection pressure on the targets in the chemical environment,
wherein the score is indicative for a worst-case coverage of the targets by the selection pressure in the chemical environment.

Embodiment 22: A method according to embodiment 20 or 21,
wherein the score takes into account a number of components in the chemical environment in determining the score of the chemical environment.

Embodiment 23: A method according to embodiment 22,
wherein the score comprises a function of a strength of the selection pressure and a coverage of the targets by the selection pressure, and a number of components in the chemical environment.

Embodiment 24: A method according to any of the preceding embodiments, wherein simulation of one or several functions of fluxes through the target reactions relative to a growth is performed,
the method comprising the following steps:

(a) Performing a first optimization of the model imposing a plurality of constraints, thereby determining a first optimization result,
wherein a constraint sets a condition defining a growth to a fixed value or in range
; wherein a constraint sets thermodynamic bounds on fluxes in the model;
wherein the first optimization of the model optimizes a sum of uptakes,
(b) Performing a second optimization using the first optimization result, thereby determining a second optimization result,
wherein a constraint sets a growth to a fixed value or in range;
wherein a constraints sets a sum of uptakes to the first optimization result,
wherein the second optimization result is indicative for the suitability
of the chemical environment,
wherein the second optimization of the model promotes a condition identified as optimizing a sum of up-regulation targets, and
wherein the second optimization of the model promotes a condition identified as optimizing a sum of down-regulation targets.

Embodiment 25: A method according to the preceding embodiments, further comprising the step:

(c) Performing a third optimisation of the model, thereby determining a third optimization result,
wherein the number of up-regulation targets which are enhanced with respect to up-regulation targets in a reference chemical environment are optimized, and
wherein the number of down-regulation targets which are suppressed with respect to down-regulation targets in a reference chemical environment are optimized.

Embodiment 26: A method according to embodiments 24 und 25, wherein the score is given by the value

$$value = W_c \cdot \frac{(n - coverage)}{n} + W_s \cdot \frac{(1000 \cdot u - strength)}{n} + W_m \cdot c \qquad ,$$

wherein

- n denotes the number of targets,
- coverage denotes the minimal value of targets obtained in step (c),
- u denotes the number of up-regulation targets,

- strength denotes the sum of the minimised sum of up-regulation targets and the maximised sum of down-regulation targets obtained in step (b), and
- c denotes the number of components in the chemical environment,
- $W_c$, $W_s$, and $W_m$ denote mathematical weights.

Embodiment 27: A method according to any one of the preceding embodiments,
wherein the method is used for fitness scoring in a genetic algorithm.

Embodiment 28: A method according to embodiment 27,
wherein the chemical environment represents an individual in the genetic algorithm, wherein the compounds of the chemical environment represent properties or genes of an individual in the genetic algorithm.

Embodiment 29: A method according to any one of the preceding embodiments,
wherein the chemical environment is a natural environment for a cell or organism.

Embodiment 30: A method for choosing a site to isolate a strain according to embodiment 29,
wherein the method leads to an isolation of a cell or organism with a metabolic trait.

Embodiment 31: A method according to any one of the preceding embodiments,
wherein the metabolic trait leads to the production of at least one desired product by the cell or organism.

Embodiment 32: A method according to embodiment 31,
wherein the at least one desired product is a food compound, preferably an aroma compound.

Embodiment 33: A method according to embodiment 32,
wherein the at least one desired product is a polymer, an acid, an alcohol or an ester.

Embodiment 34: A method according to any one of the preceding embodiments,
wherein the cell or organism is of industrial relevance.

Embodiment 35: A method according to any one of the preceding embodiments,
wherein the cell or organism is a microorganism selected from the group of bacteria, yeast or molds.

Embodiment 36: A method according to embodiment 35,
wherein the yeast is *Saccharomyces cerevisiae.*

Embodiment 37: A method according to any one of the preceding embodiments,
wherein the chemical environment is a culture medium for the cell or organism.

Embodiment 38: A method according to embodiments 1 to 37,
wherein the chemical environment is an evolution chemical environment used to evolve the metabolic trait of the cell or organism and differs from a target chemical environment used for culturing the cell or organism to produce at least one desired product.

Embodiment 39: A method according to embodiments 1 to 37,
wherein the cell is a tumour cell.

Embodiment 40: A method according to embodiments 1 to 37,
wherein the organism is a pathogen.

Embodiment 41: A method according to embodiments 39 or 40,
wherein the metabolic trait leads to the destabilization of the cell or organism.

Embodiment 42: A method according to embodiment 41,
wherein the destabilization of the cell or organism causes decreased growth and/or death of the cell or organism.

Embodiment 43: A method for determining a chemical environment to evolve a metabolic trait of a cell or organism comprising the following steps:

(a) Evaluating the suitability of at least two chemical environments using the method to any one of the preceding embodiments,
(b) Selecting at least one chemical environment with a suitability to evolve a metabolic trait, which exceeds a pre-set threshold of suitability.

Embodiment 44: A method for evolving a metabolic trait of the cell or organism comprising the steps of embodiment 43 and further comprising the step,

(c) Growing the cell or organism in the chemical environment selected in step (b).

Embodiment 45: Cell or organism obtained by the method of embodiment 44.

Embodiment 46: A method for evolving a metabolic trait of the cell or organism of embodiment 44 comprising the steps,

(a) Determining an evolution chemical environment to evolve the metabolic trait of the cell or organism according to steps (a) and (b) of embodiment 43,
(c) Growing the cell or organism in the evolution chemical environment to evolve the desired trait,
(d) Growing the cell or organism in a target chemical environment to produce at least one desired product by the cell or organism.

Embodiment 47: A computer program element, which, when executed by a processor, is adapted to carry out the method steps according to any of the embodiments 1 to 43.

Embodiment 48: A computer readable medium, comprising a computer program element according to embodiment 47.

**Claims**

1. A method for evaluating the suitability of a chemical environment to evolve a desired metabolic trait of a cell or organism, the method comprising simulation of one or several of functions of fluxes in a metabolic model,
wherein the metabolic model comprises a stoichiometric representation of biochemical reactions and import and export of extracellular compounds,
wherein the metabolic trait comprises a set of targets,
wherein the targets are functions of fluxes.

2. A method according to claim 1, wherein simulation of one or several targets relative to growth is performed,
wherein the growth is a function of fluxes through a reaction or reactions that generate biomass components or biomass and wherein a simulation is performed while the growth is constrained to a fixed value or within a range.

3. A method according to claim 2, wherein simulation is performed while a constraint is set on any of uptakes,
wherein uptakes are functions of fluxes through the reactions representing the import of extracellular compounds.

4. A method according to claim 3, wherein a simulation is performed while a sum of uptakes is constrained to a fixed value or within a range, wherein optionally a simulation is performed while a sum of uptakes is constrained to an optimal value, and/or wherein a simulation is performed while any of uptakes is constrained to a fixed value or within a range.

5. A method according to any of the preceding claims, wherein up-regulation targets and down-regulation targets are optimized into opposite directions,
wherein the up-regulation targets are those targets for which an increase is desired, and
wherein the down-regulation targets are those targets for which a decrease is desired,
wherein optionally absolute values of up-regulation targets are minimized and absolute values of down-regulation targets are maximized, and/or wherein the number of targets exceeding or falling below at least one threshold is optimized.

6. A method according to any of the preceding claims,
wherein the number of targets relative to a growth exceeding or falling below at least one threshold is simulated and wherein optionally the at least one threshold is determined with respect to functions of fluxes in a reference chemical environment.

7. A method according to any of the preceding claims, wherein in the simulation at least one inhibited flux is defined, and wherein optionally simulation is performed by constraining to zero fluxes through reactions that are targets of inhibitors or regulatory triggers included in the chemical environment.

8. A method according to any of the preceding claims, further comprising:

   determination of a numerical score for the chemical environment,

   wherein the score is indicative for a strength of a worst-case selection pressure on the targets in the chemical environment,
   wherein the score is indicative for a worst-case coverage of the targets by the selection pressure in the chemical environment, and/or wherein the score takes into account a number of components in the chemical environment in determining the score of the chemical environment.

9. A method according to any of the preceding claims, wherein simulation of one or several functions of fluxes through the target reactions relative to a growth is performed,
   the method comprising the following steps:

   (a) Performing a first optimization of the model imposing a plurality of constraints, thereby determining a first optimization result,
   wherein a constraint sets a condition defining a growth to a fixed value or in range;
   wherein a constraint sets thermodynamic bounds on fluxes in the model;
   wherein the first optimization of the model optimizes a sum of uptakes,
   (b) Performing a second optimization using the first optimization result, thereby determining a second optimization result,
   wherein a constraint sets a growth to a fixed value or in range; wherein a constraints sets a sum of uptakes to the first optimization result,
   wherein the second optimization result is indicative for the suitability of the chemical environment,
   wherein the second optimization of the model promotes a condition identified as optimizing a sum of up-regulation targets, and
   wherein the second optimization of the model promotes a condition identified as optimizing a sum of down-regulation targets.

10. A method according to the preceding claims, further comprising the step:

    (c) Performing a third optimisation of the model, thereby determining a third optimization result,
    wherein the number of up-regulation targets which are enhanced with respect to up-regulation targets in a reference chemical environment are optimized, and
    wherein the number of down-regulation targets which are suppressed with respect to down-regulation targets in a reference chemical environment are optimized.

11. A method according to claims 9 and 10,
    wherein the score is given by the value

$$value = W_c \cdot \frac{(n-coverage)}{n} + W_s \cdot \frac{(1000 \cdot u - strength)}{n} + W_m \cdot c \qquad ,$$

    wherein

    - n denotes the number of targets,
    - coverage denotes the minimal value of targets obtained in step (c),
    - u denotes the number of up-regulation targets,
    - strength denotes the sum of the minimised sum of up-regulation targets and the maximised sum of down-regulation targets obtained in step (b), and
- c denotes the number of components in the chemical environment,
- $W_c$, $W_s$, and $W_m$ denote mathematical weights.

12. A method for determining a chemical environment to evolve a metabolic trait of a cell or organism comprising the following steps:

   (a) Evaluating the suitability of at least two chemical environments using the method to any one of the preceding claims,
   (b) Selecting at least one chemical environment with a suitability to evolve a metabolic trait, which exceeds a pre-set threshold of suitability.

13. A method for evolving a metabolic trait of the cell or organism comprising the steps,

   (a) Determining an evolution chemical environment to evolve the metabolic trait of the cell or organism according to steps (a) and (b) of claim 12,
   (c) Growing the cell or organism in the evolution chemical environment to evolve the desired trait,
   (d) Growing the cell or organism in a target chemical environment to produce at least one desired product by the cell or organism.

14. Cell or organism obtained by the method of embodiment 13.

15. A computer readable medium, comprising a computer program element, which, when executed by a processor, is adapted to carry out the method steps according to any of the embodiments 1 to 11.

Figure 1

Figure 2

Figure 3

|       | Comp 1 | Comp 2 | Comp 3 | Comp 4 | . | . | . |
|-------|--------|--------|--------|--------|---|---|---|
| Ind 1 | 1      | 0      | 0      | 1      |   |   |   |
| Ind 2 | 0      | 0      | 1      | 1      |   |   |   |
| Ind 3 | 0      | 1      | 1      | 0      |   |   |   |
| Ind 4 | 0      | 1      | 0      | 0      |   |   |   |
| .     |        |        |        |        |   |   |   |
| .     |        |        |        |        |   |   |   |
| .     |        |        |        |        |   |   |   |

|       |   |   |   |   |   |   |   |
|-------|---|---|---|---|---|---|---|
| Ind 2 | 0 | 0 | 1 | 1 | . | . | . |

$$fitness = f(Ind)$$

| Crossover |   |   |   |   |   |   |   |
|-----------|---|---|---|---|---|---|---|
| Ind A     | 1 | 0 | 0 | 1 | . | . | . |
| Ind B     | 0 | 0 | 1 | 1 | . | . | . |

|       |   |   |   |   |   |   |   |
|-------|---|---|---|---|---|---|---|
| Child | 1 | 0 | 1 | 1 | . | . | . |

| Mutation |   |   |   |   |   |   |   |
|----------|---|---|---|---|---|---|---|
| Child    | 1 | 0 | 1 | 0 | . | . | . |

**+**

| Best scoring x % |   |   |   |   |   |   |   |
|------------------|---|---|---|---|---|---|---|
| Ind I            | 0 | 0 | 1 | 1 | . | . | . |
| Ind II           | 0 | 1 | 1 | 0 | . | . | . |
| Ind III          | 0 | 1 | 0 | 0 | . | . | . |
| .                |   |   |   |   |   |   |   |
| .                |   |   |   |   |   |   |   |
| .                |   |   |   |   |   |   |   |

|       | Comp 1 | Comp 2 | Comp 3 | Comp 4 | . | . | . |
|-------|--------|--------|--------|--------|---|---|---|
| Ind 1 | 0      | 0      | 0      | 1      |   |   |   |
| Ind 2 | 1      | 1      | 0      | 1      |   |   |   |
| Ind 3 | 1      | 0      | 1      | 0      |   |   |   |
| Ind 4 | 1      | 0      | 0      | 0      |   |   |   |
| .     |        |        |        |        |   |   |   |
| .     |        |        |        |        |   |   |   |
| .     |        |        |        |        |   |   |   |

**Generation of an initial population**
Set of individuals each defining a random combination of compounds (i.e. a chemical environment)

quit?

**Fitness scoring of individuals**

**Reproduction**

Crossover

Mutation

Elitisism
i.e. carry over of best individuals

New generation

Europäisches
Patentamt

European
Patent Office

Office européen
des brevets

**EUROPEAN SEARCH REPORT**

Application Number

EP 16 17 8113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BALÁZS SZAPPANOS ET AL: "Adaptive evolution of complex innovations through stepwise metabolic niche expansion", NATURE COMMUNICATIONS, vol. 7, 20 May 2016 (2016-05-20), page 11607, XP055329971, DOI: 10.1038/ncomms11607 * whole document, in particular title, abstract, p. 7, 8 * | 1-15 | INV. G06F19/12 |
| X | Balázs Papp ET AL: "A critical view of metabolic network adaptations", HFSP Journal, 1 February 2009 (2009-02-01), pages 24-35, XP055329972, France DOI: 10.2976/1.3020599 Retrieved from the Internet: URL:https://www.ncbi.nlm.nih.gov/pmc/articles/PMC2689614/pdf/HJFOA5-000003-000024_1.pdf [retrieved on 2016-12-16] | 1 | |
| A | * whole, document, in particular p. 29, table II * | 2-15 | TECHNICAL FIELDS SEARCHED (IPC) G06F |
| X | WO 02/061115 A2 (UNIV CALIFORNIA [US]) 8 August 2002 (2002-08-08) | 1 | |
| A | * whole document, in particular claim 1, example 2 * | 2-15 | |

The present search report has been drawn up for all claims

| Place of search | Date of completion of the search | Examiner |
|---|---|---|
| Munich | 10 January 2017 | Lüdemann, Susanna |

EPO FORM 1503 03.82 (P04C01)

## ANNEX TO THE EUROPEAN SEARCH REPORT
## ON EUROPEAN PATENT APPLICATION NO.

EP 16 17 8113

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2017

| Patent document cited in search report | | Publication date | Patent family member(s) | | Publication date |
|---|---|---|---|---|---|
| WO 02061115 | A2 | 08-08-2002 | CA | 2434968 A1 | 08-08-2002 |
| | | | EP | 1377901 A2 | 07-01-2004 |
| | | | EP | 2226739 A1 | 08-09-2010 |
| | | | JP | 2004520829 A | 15-07-2004 |
| | | | JP | 2008178415 A | 07-08-2008 |
| | | | JP | 2010157249 A | 15-07-2010 |
| | | | US | 2002142321 A1 | 03-10-2002 |
| | | | US | 2007016383 A1 | 18-01-2007 |
| | | | US | 2007055457 A1 | 08-03-2007 |
| | | | US | 2008176327 A1 | 24-07-2008 |
| | | | US | 2012021522 A1 | 26-01-2012 |
| | | | WO | 02061115 A2 | 08-08-2002 |

EPO FORM P0459

For more details about this annex : see Official Journal of the European Patent Office, No. 12/82